# EUROPEAN PATENT SPECIFICATION

(11) **EP 4 137 106 B1**
(45) Date of publication and mention of the grant of the patent: **25.09.2024**
(21) Application number: 21192147.3
(22) Date of filing: 19.08.2021
(51) Int. Cl.: A61F 13/02

(54) **ANALYTE MONITORING SYSTEM AND DEVICE COMPRISING A BODY PART THAT IS ATTACHABLE TO A BODY OF A PATIENT**
ANALYTÜBERWACHUNGSSYSTEM UND VORRICHTUNG UMFASSEND EINEN KÖRPERTEIL, DER AN EINEM KÖRPER EINES PATIENTEN ANBRINGBAR IST
SYSTÈME DE SURVEILLANCE D'ANALYTE ET DISPOSITIF COMPRENANT UNE PARTIE DE CORPS POUVANT ÊTRE FIXÉE SUR LE CORPS D'UN PATIENT

(43) Date of publication of application: 22.02.2023
(60) Divisional application: 24193247.4
(73) Proprietor: Roche Diabetes Care GmbH, 68305 Mannheim (DE); F. Hoffmann-La Roche AG, 4070 Basel (CH)
(72) Inventor: Roscher, Olaf, 68305 Mannheim (DE)
(74) Representative: Grünecker Patent- und Rechtsanwälte PartG mbB

(56) References cited:
- US-A- 5 728 071
- US-A1- 2008 119 775
- US-B1- 6 524 284
- US-B1- 7 129 389

## Description

The present disclosure relates to a device comprising a body part that is attachable to the skin of a patient according to claim 1 and an analyte monitoring system comprising such a device according to claim 11, as well as a method according to claim 13 and a kit comprising a device, a functional element and an inserting device according to claim 15.

### Prior Art

Analyte monitoring systems, like for example glucose monitoring systems have been known for a long time. Some of these systems are provided so that a patient can handle its glucose measurements on their own.

For that purpose, nowadays glucose measurement systems usually comprise, among other components, an analyte monitor with which the patient can interact and which, for example, may comprise processing circuitry, a display, input means and the like. Furthermore, nowadays analyte monitoring systems comprise a transcutaneous sensor system that may comprise two components. A first component is or comprises a body part that is attachable to the skin of a patient and has a receiving opening through which, for example by means of a needle, a sensor means, like an electrode or a plurality of electrodes or other sensing means can be inserted into the body in a subcutaneous position. The sensor means may be understood as a second component of the transcutaneous sensor system. The body part is also known as a patch and can comprise additional components, like an energy source for providing energy to the sensor means or a transmitter for transmitting data to the analyte monitoring system.

The introduced subcutaneous sensor means are then connectable to the analyte monitoring system for transferring analyte data, for example data indicative of a glucose value of the patient. The analyte monitor can, for example by means of its processing circuitry or internal programming, then calculate the actual glucose level of the patient and, for example, provide an output to the patient. This output can, for example, comprise the actual glucose value or warnings regarding hyper- or hypo-glycemia.

As the transcutaneous sensor system requires introduction and/or removal of the subcutaneous sensor means through the body of a patient, this causes an open wound in the patient's body which may constitute an infection risk. Furthermore, a body liquid like blood, interstitial fluid or sweat may leak out of the open wound and may potentially ingress into the analyte monitor or even appear on the outside of the analyte monitor which is inconvenient for the user and may also cause malfunctioning of the transcutaneous sensor system. Moreover, the used subcutaneous sensors are highly sensitive which can result in negative impact when accidentally being subject to a current flow or high voltages. This can, for example, happen when an accidentally "charged up" person touches the person wearing the transcutaneous sensor system at the place where the transcutaneous sensor system is placed at the body of the patient or when body liquid ingress into the analyte monitor leads to a short circuit. This can damage the sensor means and in extreme cases can cause failure of the sensor means. Such shortcomings associated with the available sensor systems also apply to other known medical devices comprising a transcutaneously placed functional element such as body worn drug infusion pumps including patch pumps that are equipped with a drug infusion cannula which is inserted into the skin.

Document US7129389B1 discloses an elastomeric puncture site patch for adhering to skin and antiseptically covering the skin at an area where a sharp object such as a syringe needle or intravenous is to be inserted. The patch is comprised of an elastomeric transparent self-sealing membrane and a spacer having an aperture. The spacer has an adhesive film on the surface opposite the membrane for adhering the patch to the skin. When the patch is applied to an area of the skin, a chamber is formed between the skin and the membrane and bounded by the spacer. In use, the skin is antiseptically cleaned and the patch applied. The syringe needle or similar device punctures the membrane and the skin. At the end of the procedure the needle is withdrawn and any blood remaining on the outside of the needle or oozing from the wound is trapped in the chamber, thus preventing contamination of the area beyond the chamber. A method of manufacturing the puncture site patch is also provided.

Document US5728071A discloses an injection patch for facilitating injection into a patient and for confining blood from the injection wound, which has an absorbent pad against the skin of a patient around an intended injection site and a second surface. An elastomeric, self-sealing membrane through which an injection needle can penetrate lies against the second surface of the pad and closes the central opening. A cover layer with a central opening overlies the membrane and exposing a central portion of it to identify the injection site. The patch is adhesively held to the patient's skin. An injection can be made with a needle passed through the membrane and the patient's skin, after which the needle is extracted while the membrane wipes the needle, reseals and forms a cavity with the pad and the patient's skin to contain blood from the injection site until after hemostasis.

Document US6524284B1 relates to a self adhering patch which is positioned upon the needle of a syringe. When the skin of the patient is pierced with the hypodermic needle the patch comes into contact with the skin, self adhering to the skin. As the needle is withdrawn from the patient, the patch remains upon the skin of the patient and serves as a seal against the expulsion of blood or other body fluids from the injection site.

Document US2008/119775A1 discloses a self-closing antiseptic plaster for puncturing blood vessels, muscle tissue, tissue, skin, organs (biopsy) or bone marrow, which is puncturable by cannulas, needles or the like. It has a backing layer, a sealing layer made of a material having an elastic restoring force and a skin adhesive layer applied to the underside U of the backing layer, said the sealing layer being applied to the backing layer by means of an adhesive layer. The backing layer is usually thin. The backing layer, the sealing layer, the adhesive layer and the skin adhesive layer are transparent or approximately transparent in the entire area or at least in a superimposed partial area. The backing layer and the sealing layer are made of thin and soft materials so that any puncture site is visible and/or palpable through the self-closing antiseptic plaster. The advantages include the fact that the antiseptic state at the puncture site is preserved before, during and after the puncturing.

Therefore there is a need to provide a device for inserting a functional element that avoids the shortcomings in safety and hygiene described above.

### Object

An object of the present disclosure therefore relates to providing a device for inserting a functional element into a body of the patient that can prevent a body fluid including blood, interstitial fluid, and sweat from flowing out of a receiving opening or from coming into contact with or entering components (for example electrical components) of the body part or prevent the functional element inserted into the body of the patient through the receiving opening from being subjected to unintended current flows.

One or more of the above objects are solved by the device comprising a body part that is attachable to a body of a patient according to independent claim 1, and the analyte monitoring system comprising a transcutaneous sensor system and an analyte monitor connected to the transcutaneous sensor system in line with claim 11, as well as the method for retracting an inserting device from a receiving opening in line with claim 13, and a kit comprising a device, a functional element and an inserting device according to claim 15.

Further preferred embodiments are provided in the dependent claims.

According to one embodiment, a device comprising a body part that is attachable to a body of a patient is provided, wherein the body part comprises a receiving opening through which a functional element can be reversibly introduced into a body of a patient by means of an inserting device, like a needle, wherein the body part comprises a sealing member that is adapted to seal at least a part of the receiving opening after removal of the inserting device. The needle may in some embodiments at least partially or completely envelop the functional element at least during insertion of the functional element into the skin. In other embodiments, for example where the functional element is a cannula of a drug delivery system, the needle may also act as a guiding needle and may be introduced into the cannula for guiding the cannula into the body of the patient.

The device and the other embodiments of the invention are associated with the advantage that a sealing member which is adapted to seal at least a part of the receiving opening after removal of the inserting device prevents body fluids emerging from the wound from passing the sealing member With this, also current shortcuts can be prevented and appearance of body fluids outside the device can be avoided. This way, the safety of the device is increased as malfunction of the device can be avoided. The device of the invention, according to some embodiments, also improves the hygienic situation as no body fluid will emerge from the device due to the sealing member. Preferably, the sealing member also decreases the chance of infection at the wound site as the sealing member prevents or significantly decreases the chance that germs, dirt, etc. present around the device pass through the seal into the wound.

The device may, in some embodiments, form part of a transcutaneous sensor system. In this context, the functional element may be realized as a subcutaneous sensor that is implemented in the body of a patient or introduced into the body of the patient through the body part of the device. The device itself may be connectable or connected to an analyte monitor like a glucose analyte monitor, but may, in itself, not comprise further active components like processing logic or display devices. In some embodiments, however, the device may additionally comprise an energy source, for example for providing energy to a functional element. Additionally or alternatively, a transmitter or a transceiver may be part of the device for receiving and/or transmitting data obtained from the functional element for example to an analyte monitor. The device may further comprise electrically conductive contacts that can be connected to an analyte monitor.

The device may, in other embodiments, form part of a transcutaneous drug infusion pump system. In this context, the functional element may be realized as a subcutaneous infusion cannula for administering a drug into a patient, wherein the subcutaneous infusion cannula is implanted, in use, in the body of the patient or introduced into the body of the patient through the body part of the device.

In some embodiments the functional element may be a subcutaneous sensor, a subcutaneous drug infusion cannula or a combined subcutaneous drug infusion cannula with a subcutaneous sensor. In some embodiments the device comprises a body part which is a pump housing comprising one or more of a base plate for attaching the body part to the skin of the patient, a reservoir for storing the drug, a fluid path connecting the reservoir with the subcutaneous infusion cannula, a pump drive, an energy source and a pump transmitter which controls the drug infusion and which communicates with a remote monitor. As described in other parts of this document, in the context of the transcutaneous drug infusion pump system, the pump housing comprises a receiving opening through which a functional element can be reversibly introduced into a body of a patient by means of an inserting device, like a needle, wherein the body part comprises a sealing member that is adapted to seal at least a part of the receiving opening after removal of the inserting device. The needle may, in some embodiments, at least partially or completely envelop the functional element at least during insertion of the functional element into the skin. In an embodiment, the subcutaneous infusion cannula is made of a flexible material such as plastic.

By providing a sealing member that is configured to seal at least a part of the receiving opening after the removal of the inserting device, the functional element introduced into the body of the patient through the receiving opening can be protected against external influences like currents while, at the same time, being preferably able to prevent blood or other body fluids from flowing out of the device or to come into contact with critical components of the device through the receiving opening or to prevent external agents like dirt, germs located outside of the device of the invention to reach the wound. The sealing member can be realized as a "self-acting" sealing member that performs the necessary actions for sealing the at least part of the receiving opening. Thereby, the patient is relieved from having to take care of outflowing blood on their own while, during everyday use, the risk of damaging the functional element or having the wound infected is reduced significantly.

It can be provided that the sealing member comprises a self-sealing material that is adapted to seal or partially seal an opening in the self-sealing material that was caused by an insertion device, when or after the inserting device is removed.

In the context of the present disclosure, the term "self-sealing material" means that the material, by means of its material properties and/or its geometrical structure and/or other characteristics, seals, without further intervention of the user (like a patient) or other components acting on the self-sealing material apart from the insertion device, the opening caused by the insertion device in the self-sealing material. This is intended to encompass that some interaction with the insertion device may occur and, upon this interaction, the self-sealing materials cause a sealing of the opening caused by the inserting device in the self-sealing material.

This is further intended to mean that the insertion device is inserted into the receiving opening and thereby passes the self-sealing material which is, for that purpose, preferably already placed in the receiving opening before introducing the inserting device into the receiving opening for placing the functional element. With this embodiment, the ease of use is further improved for the patient as the patient does not need to take care of the sealing of the receiving opening after retracting the inserting device.

In one embodiment, the self-sealing material comprises a foil or a foam or a preformed material.

The foil and/or the foam can comprise or can be made from, for example, a plastic material and/or may comprise polymers. A punctuation in the foil or the foam caused by the inserting device can reliably be sealed as both the foil and the foam are preferably flexible in shape. Moreover, a foam can advantageously interact with body fluids like blood, thereby, in some embodiments, extending in dimension and automatically sealing the opening caused by the inserting device when the inserting device is removed.

A pre-formed material may, for example, comprise or may be made of rubber that, by virtue of its form or internal stress that may be additionally introduced, causes an extension of the material into the hole caused by the insertion device.

In another embodiment, the sealing member comprises a sealing element that can be introduced into the receiving opening. This may, for example, encompass liquid materials that (preferably automatically) flow into the sealing opening when the inserting device is removed or retracted. Also already solidified materials like pre-formed rubber elements can be used in this embodiment.

More specifically, it can be provided that the sealing element comprises a stopper that can adapt to the form of the receiving opening.

The stopper can, for example, be made of rubber and can reliably seal the receiving opening. When the stopper is made of rubber or a corresponding material that is not electrically conductive, such a sealing member can additionally protect the functional element against current or high voltages.

In one embodiment, the sealing member comprises a movable element that can be moved, by an inserting device, into an open position when the inserting device is inserted through the receiving opening and into a closed position when the inserting device is retracted from the receiving opening.

The movable element can preferably be pre-tensioned in its closed position, for example by means of a spring element or by material properties of the movable element. For example, the movable element can be of an elastic piece of polymer or other material that can be moved by the inserting device when the inserting device is introduced into the receiving opening with some force in order to penetrate the tissue below, but moves into its pre-tensioned position (which may be the closed position) once the inserting device is retracted from the receiving opening.

Additionally or alternatively, it may also be provided that the movable element is magnetic or in any other way provided to interact with the inserting device so that when retracting the inserting device, a force acts on the movable element that causes the movable element to move together with the inserting device in the retracting direction, thereby closing the opening.

With this movable element, the forces acting upon the needle during insertion of the functional element can be reduced, thereby reducing the risk of injuries to the patient or discomfort of the patient when introducing the functional element. Additionally or alternatively, this embodiment may provide some guiding of the insertion needle, thereby aiding also unexperienced patients in correctly inserting the functional element.

In a further embodiment, the sealing member comprises an absorbent element that is adapted to absorb a fluid and change, upon absorbing a fluid, its form so as to seal at least part of the receiving opening after removal of an inserting device.

The absorbent element can, for example, be or comprise a foam or other material that preferably increases its size when getting into contact with fluids it can absorb. Thereby, the space occupied by the absorbent element increases when being in contact with body fluids, thereby causing the receiving opening to close after removal of the inserting device.

More specifically, the absorbent material may comprise components that support coagulation of blood. Closing of the wound of the patient's wound is thereby supported further, thus reducing the risk of infections.

In a further embodiment, the sealing member is made from or comprises an electrically insulating material so that a flow of electrical current through the receiving opening is prevented when the sealing member seals at least a part of the receiving opening after removal of the inserting device.

According to the invention, the sealing member is made from or comprises an electrically conductive material and the electrically conductive material is conductively connectable to the skin of a patient when the device is attached to the body of a patient.

The sealing member does not need to be fully made of the electrically insulating material, but it may be sufficient, in some embodiments, if a coating is provided that protects the interior of the receiving opening below the sealing member (i.e. in directions closer to the patient's skin) against current or high voltages. With this alternative, currents can be prevented from entering into the receiving opening and the subcutaneous region in which the functional element is arranged, thereby preventing damage to the functional element.

Providing the sealing member with an electrically conductive material does also not necessarily require that the sealing member is completely made from the electrically conductive material but a coating may be provided. The conductive connectability to the skin of a patient can be realized for example by means of a cable or the other structural characteristics of the device. Thereby, the current that is received at the sealing member in the receiving opening can be guided to the surrounding skin of the patient. Damage to the functional element can thus be avoided.

In one embodiment, a combination of a functional element and a device according to any of the above embodiments is provided, wherein the functional element is or comprises a subcutaneous sensor, a subcutaneous drug infusion cannula or a combined subcutaneous drug infusion cannula with a subcutaneous sensor.

Moreover, an analyte monitoring system comprising a transcutaneous sensor system and an analyte monitor connected to the transcutaneous sensor system, the transcutaneous sensor system comprising a device according to any of the above embodiments, a subcutaneous sensor and a transmitter, wherein the subcutaneous sensor is adapted to sense presence of at least one analyte and wherein the transmitter is adapted to transmit a signal indicative of a sensed analyte to the analyte monitor.

The analyte monitor may be directly or indirectly connected to the subcutaneous sensor, for example, by means of a wired or wireless connection to the subcutaneous sensor or indirectly through the device according to the above embodiments. For example, the body part of the device of the above embodiments may comprise electrodes or other components for connecting to the analyte monitor.

This analyte monitoring system is easy to use by a patient and ensures protection of its sensor as functional element against environmental influences. Furthermore, blood is prevented from flowing out of the receiving opening, thereby also avoiding damage to the analyte monitor or its contacts.

In a more specific embodiment, the analyte monitoring system is a continuous analyte monitor and the subcutaneous sensor is adapted to sense glucose in a body fluid of a patient when implanted. The above-mentioned advantages of the invention are thereby provided for systems used to monitor the glucose level of a patient.

Moreover, a method is provided, the method comprising retracting an inserting device from a receiving opening of a body part of a device, wherein the body part is attached to the body of a patient, wherein the method further comprises sealing at least part of the receiving opening with a sealing member of the body part after retracting the inserting device from the receiving opening.

With this method, closing of the receiving opening is ensured after the inserting device is retracted.

In one embodiment, the method comprises, before retracting the inserting device from the receiving opening, inserting the inserting device through the receiving opening and placing a functional element into the body of the patient.

Placement of a functional element, like a subcutaneous sensor for detecting an analyte in a body fluid, can thereby be provided in a reliable manner.

Specifically, the device may be a device according to any of the above embodiments.

Moreover, a kit is provided in some embodiments, the kit comprising a device according to any of the above embodiments, a functional element and an inserting device for inserting the functional element into the body of a patient.

This kit can be used easily by a patient for inserting the functional element, like a sensor for sensing an analyte in the body fluid of a patient, and can, for example, be sterilized so that no infectious risk is posed. The kit may also comprise an analyte monitor that is connectable to the device and/or the functional element as described in the above embodiments.

Specifically, the inserting device may comprise or may be a needle. Needles can be handled comparably easy by most users, also potentially allowing for introducing the functional element without the help of a medical professional.

### Brief description of the drawings

- Figure 1: shows an exemplary embodiment of an analyte monitoring system;
- Figures 2a-2f: show schematic depictions of the insertion of a functional element through a receiving opening of the body part of the first device in some embodiments;
- Figures 3a to 3f: show embodiments of sealing members;
- Figure 4: shows a kit according to one embodiment.

### Detailed description

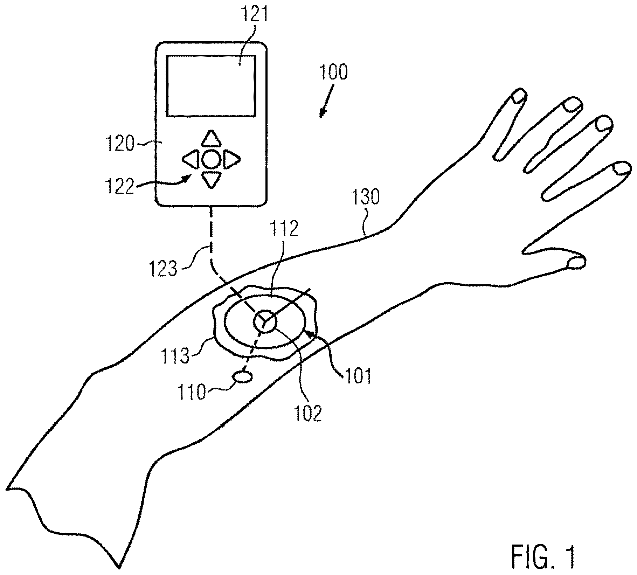

Figure 1 shows an analyte monitoring system 100 in use on an arm of a patient 130, though other body sites such as the abdomen, hip, leg, etc. are also feasible. The analyte monitoring system comprises an analyte monitor 120. This analyte monitor may be used as a display device and/or as an input device for interacting, via the user, with the analyte monitoring system.

The analyte monitor may, for that purpose, encompass not further shown processing circuitry and internal memory as well as one or more connections for data transfer. These connections for data transfer may, in some embodiments, comprise at least one connection 123 for connecting the analyte monitor 120 to functional element 110. Moreover, the analyte monitor 120 may comprise a display 121 and/or one or more keys 122 for providing input and/or interaction with the user.

Moreover, the analyte monitor 120 may comprise an energy source like batteries or accumulators that are rechargeable and allow the analyte monitor to be powered with energy preferably over a long period of time of several hours or days so that a patient using the analyte monitor does not need to change the batteries or load the analyte monitor too often.

The analyte monitoring system 100 further comprises a device 101 comprising a body part 112 that can be attached to the body of the patient, specifically to the skin of the patient as in the embodiment shown in Figure 1. Attaching the body part 112 to the skin or generally the body of the patient may comprise, for example, fixing it by means of a fixation device 113 to the body of the patient. The fixation device 113 for attaching the body part 112 to the skin of the patient may be realized as an adhesive plaster but also other realizations such as a bracelet, etc. may be used. The fixation device may form part of the device 101 or may additionally be provided.

The body part can be a passive body part that does not comprise any electrical components. However, in some embodiments, it can be provided that the body part 112 comprises an energy source and/or a transmitter (or a transceiver) for transmitting signals. For example, the body part can comprise a transceiver for exchanging data with the analyte monitor 120, specifically for providing data from the functional element 110 to the analyte monitor and/or for receiving data from the analyte monitor 120.

The body part comprises a receiving opening 102 through which the functional element 110, specially an analyte sensor for sensing of an analyte like, for example, glucose, can be introduced into the body of the patient. The functional element 110 may generally be provided as a subcutaneous sensor 110. Together with the body part, the functional element 110 may, in this case, be considered to constitute a transcutaneous sensor.

Instead of a functional element that is suitable for detecting glucose or other analyte(s), also other functional elements that are not necessarily adapted to sense an analyte but to, for example, infuse drugs or medications can be inserted through the receiving opening. Accordingly, in some embodiments the functional element 110 is a subcutaneous sensor, a subcutaneous drug infusion cannula or a combined subcutaneous drug infusion cannula with a subcutaneous sensor.

The receiving opening 102 preferably has a size that is larger in diameter than the size of an inserting device with which the functional element can be inserted into the skin of the patient. In view of this, the minimum diameter extension of the receiving opening (in case it is provided as having a cross-section of a circle) is the extension of the largest extension of the functional element in particular, in cases where the insertion device is at least partially enveloped by the functional element, e.g. when the functional element is a subcutaneous drug infusion cannula which envelops an insertion needle during insertion. This largest extension may be the largest extension the functional element has or it may be the largest extension of the functional element perpendicular to an insertion direction of the functional element into the body. For example, in case the functional element is or comprises one or a plurality of electrodes in the form of small cylinders, the receiving opening may have a diameter that is at least the diameter of the functional elements increased by an amount Δ that corresponds to twice the wall size of an inserting device in case the inserting device is realized as a needle.

Alternatively, if the functional element is or comprises a subcutaneous sensor which is enveloped by the insertion device during insertion, the minimum diameter extension of the receiving opening (in case it is provided as having a cross-section of a circle) is the extension of the largest extension of the insertion device. In this case the largest extension may be the largest extension the insertion device has or it may be the largest extension the insertion device has or it may be the largest extension perpendicular to an insertion direction of the functional element into the body perpendicular to an insertion direction of the functional element into the body. The closer the diameter of the receiving opening is to this extension, the better the receiving opening can also act as a guide for guiding insertion or implantation of the functional element into the skin of the patient.

Though the above example was discussed for a receiving opening having a circular cross-section, the shape of the receiving opening is not restricted. The cross-section of the receiving opening perpendicular to the direction from an outer surface of the body part (that faces, in use of the device, away from the skin of the patient) to an inner surface that is in contact with or used for attaching the body part to the skin of a patient (i.e. a surfaces that faces, in use of the device, the skin of the patient) can, for example, also be rectangular or ellipsoidal or can have any other shape.

Along this direction, the receiving opening may have constant diameter and may therefore generally be provided in the form of a cylinder (with not necessarily circular ground and top plane as mentioned above). Alternatively, the inner diameter may increase or decrease from the outer surface to the inner surface. In the latter case, the receiving opening can act as an aid for guiding the inserting device to the actual location where the functional element is to be placed.

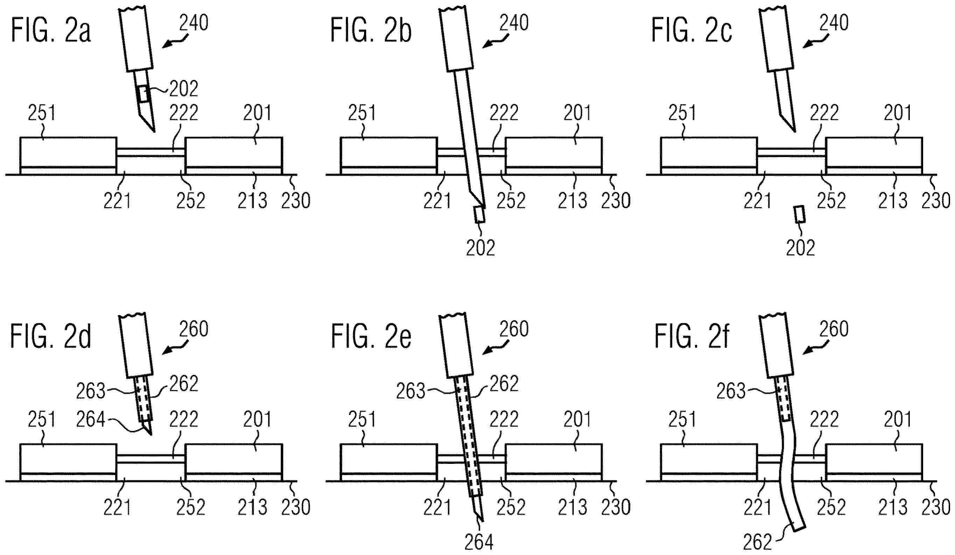

Figures 2a to 2f show a cross-sectional view of the body part 201 of the device 101 described in Figure 1 on the skin 230 of a patient.

The figures 2a to 2c show an embodiment where the functional element may be realized as a subcutaneous sensor, which will be explained below. The figures 2d to 2f show a case where the functional element may be realized as a cannula that is introduced into the body of a patient, for example for (continuous or bolus) drug delivery using a drug delivery system like a (continuous or bolus) insulin delivery pump.

As already mentioned above, the body part 201 may be fixed for example with a fixation device 213 (like a plaster) onto the skin 230 of the patient. Generally, the body part 201 or the device may also be referred to as a patch that is fixed by a plaster to the skin of a patient.

In the embodiment shown in Figure 2a, the inserting device 240, which can be or comprise an insertion needle, is shown for inserting a functional element 202 into a region below the skin 230 of the patient in a situation where the insertion needle is still placed outside of the body part 201, e.g. preferably prior to the insertion of the functional element 202. In this embodiment (the embodiment described in figures 2a to 2c), the insertion needle is a hollow needle that surrounds the functional element. This embodiment may be used in cases where the functional element is a subcutaneous sensor, like a glucose sensor. The functional element 202 may, in this embodiment, be provided within the inserting device 240. In some embodiments, the functional element may also be or comprise a flexible cannula that is placed within the inserting device for guiding the flexible cannula into the body of a patient.

Figure 2b now shows the situation where the inserting device 240 has been introduced into the body part 201 for placing the functional element 202 below the skin 230. As is seen from Figure 2b, the inserting device 240 or at least part of the inserting device like an insertion needle is introduced through the receiving opening 221 of the body part 201 into the body of the patient to place the functional element 202 below the skin 230 of the patient. The insertion needle can be slotted or unslotted. According to embodiments of the invention, the process of inserting the inserting device 240 into the body of the patient encompasses passing the inserting device with the functional element 202 through a sealing member 222 in the receiving opening 221 of the body part 201. The sealing member 222 may thereby be partially deformed or damaged as the inserting device 240 passes through it.

After passing the inserting device through the receiving opening 221 and potentially also through the sealing member 222, the inserting device 240 at least partially pierces through the skin 230 of the patient and can place, below the skin 230 of the patient, the functional element 202, which, as already mentioned above, may for example comprise an analyte sensor and may, for example, be realized as one or more electrodes. Alternatively, the functional element may comprise a cannula for continuous drug delivery.

After placing of the functional element 202 below the skin 230 of the patient, the inserting device 240 is retracted from the position where it pierces through the skin of the patient. This is shown in figure 2c. Preferably, the subcutaneously inserted functional element 202 remains in its position as the insertion device 240 retracts. In the case depicted in figures 2a to 2c, this may encompass that the analyte sensor as one realization of the functional element 202 remains in its position below the skin after having been placed there by the inserting device 240.

Embodiments of methods of the present disclosure comprise retracting, after the inserting device 240 has been retracted from inside the body of the patient, the inserting device 240 even further through the receiving opening 221. Thereby, the inserting device will also pass or leave the sealing member 222 through which it was previously pierced in a direction of the upper surface 251.

Having retracted the inserting device 240 also from the sealing member 222 in the receiving opening 221, embodiments of the present disclosure then comprise the sealing of the receiving opening 221 by means of the sealing member 222 itself, in some embodiments around the functional element 202, for example in case the functional element is realized as a cannula for drug delivery that extends, even after placement outside of the sealing member 222.

This preferably encompasses completely sealing the receiving opening, in some embodiments around the functional element 202, so that the region 252 depicted in Figure 2 between the skin 230 and the sealing member 222 is isolated by means of the sealing member from the outer environment. Other embodiments may comprise that the sealing member at least seals part of the opening, in some embodiments around the functional element 202, that was caused by the inserting device 240. For example, the inserting device 240 can be realized as an insertion needle having a diameter of some millimeters, for example 1mm or 2mm or more.

A puncture or hole in the sealing member of that size allows for body fluids like blood to flow out of the opening in the sealing member of the receiving opening 221. In some embodiments, in order to prevent this, the sealing member can be capable of closing or sealing the opening, in some embodiments around the functional element 202, caused by the inserting device 240 to an extent that the flow of blood or other body fluids though the still remaining opening in the sealing member is prevented completely or at least reduced to a significant degree like 50%, 60% or 70% or even 90% or 95% compared to a flow of body liquids through the opening in the sealing member if the sealing member would not at least partially close the opening caused by the inserting device 240.

Figures 2d to 2f show a different embodiment compared to the one described in relation to figures 2a to 2c. The embodiment shown here basically corresponds to the embodiment described in relation to figures 2a to 2c. In contrast to figures 2a to 2c, the inserting device 260 encompasses a guiding needle 263 that is guided through the interior of a hollow or at least partially hollow functional element 262. The functional element 262 in this embodiment may be a cannula through which, once placed in the body of a patient, a drug or medicine or other fluid can be delivered. For example, the cannula can be used to provide insulin from a continuous insulin pump to the patient.

In the situation shown in figure 2d, the inserting device 260 is equipped with the cannula 262 so that the guiding needle 263 is at least partially enveloped by the cannula 262 and extends at least partially through the cannula 262. Specifically, and as depicted, it may be provided that the tip 264 of the guiding needle 263 extends outside of the cannula so as to aid in piercing through the skin 230 of the patient and to pierce through the sealing member 222.

Accordingly, from the side 251 of the body part 201 that faces away from the skin 230 of the patient, when inserting the functional element of this embodiment, the cannula 262 is guided by the guiding needle 263 of the inserting device 260 through the sealing member 222 in the receiving opening 221 and through the skin 230 of the patient. This situation is shown in figure 2e. This encompasses piercing through and thereby causing an opening within the sealing member 222 so that the cannula is firstly introduced into the region 252 already explained above. Then, the cannula, optionally aided by the guiding needle 263 and specifically the tip 264 of the guiding needle 263 pierces through the skin of the patient.

Having placed the cannula 262 in the body of the patient, the guiding needle 263 can be retracted while the cannula 262 is left in place. This is shown in figure 2f. In contrast to the embodiment described in figures 2a to 2c in case the functional element is realized as a subcutaneous sensor, in the present embodiment, the functional element extends, also after placement in the body of a patient as shown in figure 2f, through the receiving opening and even through the sealing member 222.

It is noted that the embodiment of Figures 2d to 2f is also applicable to cases where the functional element 262 is or comprises a sensor. In some embodiments, the sensor may not be provided as an isolated sensor element as shown in Figures 2a to 2c as having no connection to the body part, but the sensor may comprise elements, like connecting elements or electrodes for connecting the sensor electrically to the body part, that extend beyond the receiving opening and/or the sealing member. In these embodiments, like for the functional element 262 comprising a cannula, the connecting elements (or any other portion of the sensor) may extend beyond the sealing member and/or the receiving opening also after retraction of the inserting device 260.

In such embodiments where the functional element (like a sensor or a cannula) extends also beyond the sealing member and/or the receiving opening, as depicted in Figures 2d to 2f, the inserting device 260 may be embodied as a slotted or unslotted needle that surrounds the functional element or part of the functional element. Specifically, if the inserting device is embodied as or comprises a slotted needle, it can be provided that at least parts of the functional element that extend, after placement of the functional element in the body of a patient, through the receiving opening and/or the sealing member, are held by a slotted portion of the needle. This can make introduction of the functional element and later separation of the functional element from the inserting device easier also in cases where the functional element extends, after placement, through the receiving opening and/or the sealing member. For example, only a part of the functional element that will extend through the receiving opening and/or the sealing member can be introduced or included in the slotted needle while other parts of the functional element, specifically that part that establishes a connection to the body part or, for example, a drug pump, may be still external to the slotted needle. In that case, the slotted needle advantageously guides the part of the functional element that is to be introduced into the body of the patient while allowing for simplified separation of the functional element from the needle once the needle is retracted from the receiving opening and/or the sealing member, as shown in figure 2f.

While the sealing member of figures 2d to 2f can be realized in the same way as described for the embodiment of figures 2a to 2c, the sealing member 222 now acts so as to close the opening in the sealing member 222 caused by inserting the cannula 262 using the guiding needle 263 so that the functional element 262 (the cannula in this case) is surrounded by the sealing member 222 that comes into close contact with the functional element 262, thereby closing, in interaction with the functional element, the opening in the sealing member. While the actual opening in the sealing member 222 is thus not completely closed in this embodiment, it is understood that by surrounding, in close contact, the cannula or generally a functional element 262 that extends through the sealing member 222 after placement, flowing out of body liquids like blood as well as contact of such fluids with other parts of the body part can be prevented.

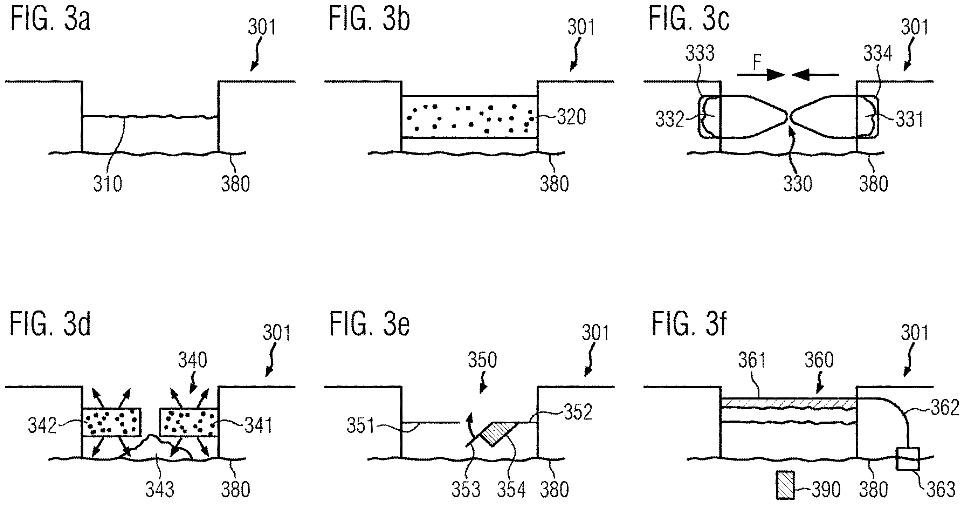

In Figures 3a to 3f different embodiments of sealing members that were, so far, only generally described in relation to Figures 1 and 2 will be described in further detail. In the Figures 3a to 3f, the sealing members are always depicted in their position in the receiving opening of the body part 301 as it is attached to the skin 380. While the figures 3a to 3f consistently show and describe a complete closing of the opening within the sealing member after retracting the inserting device, it is to be understood that this is provided only for simplicity of explanation. In embodiments where the functional element (like a cannula for drug infusion) extends also after placement through the receiving opening or at least through the sealing member (as discussed in relation to figures 2d to 2f), the described embodiments are intended to encompass that the sealing member seals the opening so that it is in close contact with the functional element that still extends through the receiving opening, thereby preventing fluids like blood from, for example, flowing outside of the receiving opening, for example along the functional element.

In the embodiment of Figure 3a, the sealing member may comprise or may be a foil 310 that is placed in the receiving opening of the body part 301 and, in the position of the body part on the skin 380 of a patient, can border a region in the receiving opening, together with the skin 380 and the interior walls of the receiving opening.

When an inserting device has been pierced through the foil 310, it can close itself for example due to frictional forces that cause moving the foil together with the inserting device when it is retracted from the receiving openings so that the damaged part of the foil, that was pierced through when inserting the inserting device, moves together with the inserting device until it is again in contact with the remaining part of the foil so that the opening in the foil caused by the inserting device is at least partially closed again. Furthermore, in some embodiments, it can be provided that the foil 310 is under some tension so that a piercing through with an inserting device, though causing a hole, does not damage the general structure of the foil and the foil remains in its position and state so that a closing of the hole within the foil caused by the inserting device at least partially occurs with the aid of those parts of the foil that have been partially loosened by the inserting device when piercing through the foil 310.

Additionally, depending on the size of the hole in the foil caused by the inserting device, the hole in the foil may be sufficiently closed or partially closed or sealed in conjunction with blood that pours out from a wound caused by the inserting device when placing the functional element under the skin of the patient. For example, when blood flows into the space below the sealing member 310, the opening in the sealing member 310 can be closed at least partially by the blood coagulating below.

The foil 310 may specifically be made from or comprise a plastic material and may be made, for example, from PET.

Figure 3b depicts a further embodiment of the sealing member in the form of or comprising a foam 320. The foam 320 can be pierced through by the inserting device, thereby causing a hole in the foam 320. Unlike in Figure 3a, this piercing though by the inserting device may cause separation of parts of the foam 320 from the remaining parts of the foam so that, when retracting the inserting device again, there is a hole in the structure of the foam 320.

In some embodiments, the foam 320 may be placed in the receiving opening with some internal pre-tension. Thereby, when introducing an inserting device through the foam and retracting it again, the internal pre-tension of the foam causes the material of the foam to move, once the inserting device has been retracted, into the opening within the foam 320 so that the hole is at least partially closed. Depending on the tension that is applied to the foam when inserting it into the receiving opening during manufacture, this process can be repeated several times allowing for reusing the sealing member of Figure 3b also several times when, for example, removing or replacing a functional element. Exchange of the body part is then not necessary for at least two cycles of reintroducing or removing or exchanging the functional element. This may be particularly advantageous in cases where the functional element is or forms part of a drug delivery system.

Preferably, the foam 320 is made from or comprises a plastic material, which, in some preferred embodiments, may even also comprise disinfecting properties. These may be provided by means of a coating or particles introduced into the foam that have a disinfecting effect. The coating or introduced particles may, for example, comprise silver ions. In one embodiment, the foam 320 may be made from or may comprise polyurethane.

In the embodiment of Figure 3c, the sealing member 330 is made from or comprises a sealing element (also denoted with 330), for example in the form of a stopper, that can be introduced into the receiving opening and can adapt to the form of the receiving opening. This sealing element can already be placed in the receiving opening before introducing the inserting device. In such a case, as in the situation of Figure 3c, the sealing element will be separated like in Figure 3b, when the inserting device is inserted through the sealing element 330. In that case, it can be preferred when at least one of the remaining parts 331 and 332 of the sealing element 330 is pre-tensioned so that, once the inserting device is removed, the parts 331 and/or 332 extend along the arrows indicated in Figure 3c due to internal forces so as to adapt to the form of the receiving opening again, thereby closing the opening in the sealing member 330.

In some embodiments, it may be provided that the body part 301 comprises one or more reservoirs 333 and 334 in which the sealing element 330 or the material of the sealing element 330 is provided and that can extend into the receiving opening as need be. This can be achieved by pre-tensioning the material in the reservoirs so that, once material of the sealing element 330 is removed when retracting an inserting device, the material moves from the reservoirs 333 and/or 334 closer into the center of the receiving opening of the body part 301.

Thereby, the sealing element 330 according to this embodiment can also be reused several times. In the reservoirs 333 and 334, the material forming the sealing element 330 may be provided in an already solidified state in the reservoirs.

Moreover, though the body part is only shown here in a cross-section, the reservoirs 333 and 334 may be provided as a single reservoir around the whole circumference of the receiving opening so that material of the sealing element can move into the receiving opening from all directions and/or extend, due to internal stress, from all directions into the center of the receiving opening once the inserting device is removed. Alternatively, several or only one separate reservoir may be provided that span or spans across a section of the circumference of the receiving opening. For example, each reservoir may not extend along more than 20% or not more than 30% or not more than 40% of the circumference of the receiving opening.

Preferred embodiments of the sealing member 330 according to Figure 3c may comprise that the sealing member is made from natural rubber or from artificial rubber material or comprises the same. Alternatively or additionally, the sealing member 330 may comprise or may consist of silicone and specifically liquid silicone rubber.

In the embodiment shown in Figure 3d, the sealing member 340 comprises an absorbent material. This can, for example, be a foam or an absorbent mesh (as can for example also be found in plasters) so that this embodiment may at least partially correspond to the embodiment of Figure 3b.

However, in the embodiment of Figure 3d, the absorbent material of the sealing member is provided so as to cause a deformation of the remaining parts 341 and 342 of the sealing member 340 when coming into contact with body fluids like blood. In some embodiments, after piercing through the sealing member 340 with the inserting device (thereby causing a hole in the sealing member) and retracting the inserting device, blood 343 from the wound in the skin 380 of the patients flows into the space below the sealing member 340 in the receiving opening. The absorbent material of the sealing member 340 then causes an extension of the sealing member (specifically the remaining parts 341 and 342) within the receiving opening preferably in all directions, thereby closing the opening caused by the inserting device and closing or sealing the receiving opening again.

For that purpose, the sealing member 340 in the embodiment of Figure 3d can be placed in the body part 301 at a position that is closer to the skin 380 compared to the position of the foam 320 in Figure 3b. Thereby, immediate or less delayed contact of blood with the absorbent material is ensured. Like for Figure 3b, the sealing member 340 of the embodiment of Figure 3d may be made from or may comprise polyurethane.

For example, the absorbent material can be placed at a distance to a lower opening of the receiving opening that will be in contact with the skin in use of the body part that is half as much as the distance to the upper opening of the receiving opening in a surface that, in use of the body part, faces away from the skin 380 of the patient. It may also be provided that the distance of the absorbent material to this lower opening is less than 2mm preferably less than 1mm. This can still ensure that no immediate contact of the absorbent material with the skin and/or the wound in the skin of the patient after removal of the inserting device occurs, while providing for reliable contact with blood pouring out from the wound. In other embodiments, it may also be provided that the absorbent material is or can be in direct contact with the skin of a patient.

Figure 3e shows a further embodiment of the sealing member where the sealing member 350 comprises a moveable element 353. This moveable element may be placed between bordering elements 351 and 352 of the sealing member 350 that extend, from the borders of the receiving opening provided by the physical structure of the body part 301 in a direction into the center of the receiving opening. These additional elements 351 and 352 may be comparably rigid so that piercing through with the inserting device may be prevented and entering the inserting device into the receiving opening may only be possible in the region where the moveable element 353 is placed.

The additional elements 351 and 352 may extend horizontally or equidistantly to the skin of a patient when the body part 301 is in use and attached to the skin 380 of the patient. In other cases, the elements 351 and 352 may be inclined towards the skin 380 of the patient so that the distance of the elements 351 and/or 352 to the skin 380, in use of the body part, is smaller for parts of the elements 351 that are closer to the center of the receiving opening. Thereby, they can act as guidance for the inserting device so that the inserting device is introduced in the receiving opening at the place of the moveable element.

The moveable element 353 is preferably realized so that it is moveable, when inserting an inserting device through the sealing member in a direction towards the skin 380, from a closed position of the moveable element 353 in which the sealing member is completely closed, to an open position that allows moving the inserting device through an in the sealing member 350.

Thereby, the inserting device moves the moveable element 353 against the direction of the arrow denoted in Figure 3e so as to pierce into the skin 380 of the patient.

When retracting the inserting device from the skin and through the receiving opening, the moveable element 353 preferably moves with the inserting device until it is in a closed position where an opening in the sealing member 350 is at least partially closed. This can, for example, be caused by friction or other forces that cause the moveable element to move with the inserting device.

In some embodiments, a pre-tensioning element 354 is provided that pre-tensions the moveable element 353 into its closed position. This can be realized, for example, by a small spring element 354 or material characteristics of the moveable element 353. For example, the moveable element can be provided as a leaf spring, where the relaxed state of the leaf spring is the closed position in which the moveable element closes the sealing member 350. Inserting an inserting device then causes a movement of this leaf spring against the spring forces, and will, when retracting the inserting device, cause the leaf spring to move again into its closed position, thereby closing the sealing member and the receiving opening.

Other embodiments may encompass a pre-tensioning of the movable element 353 into the open position. It may be provided that a corresponding pre-tensioning element that pre-tensions the movable element 353 into the open position is cut through or otherwise rendered non-functional when inserting the inserting device through the receiving opening in the body part 301. Thereby, the pre-tensioning force does no longer apply. It can be provided that the movable element 353, in such embodiments, is made from a material that is pre-tensioned into the closed position by its material characteristics. Once the pre-tensioning element 354 is rendered non-functional, no pre-tensioning force pre-tensioning the movable element 353 into the open position applies. When retracting the inserting device from the sealing member, the force pre-tensioning the movable element 353 to the closed position can cause a closing of the opening within the sealing member 350. The movable element 353 may, in this embodiment, be realized as a shape memory material made from or comprising, for example, Nitinol. The shape memory material causes a pre-tensioning into the closed position so that the movable element assumes its pre-tensioned state when the pre-tensioning element 354 is non-functional.

In this embodiment, it can be preferred that the pre-tensioning force of the pre-tensioning element 354 is larger than the pre-tensioning force of the movable element 353 itself. Instead of a pre-tensioning of the movable element 353 by its material characteristics, also an additional pre-tensioning element 354 may be provided that pre-tensions the movable element into its closed position where preferably the pre-tensioning force of the pre-tensioning element pre-tensioning the movable element 353 in its open position is again larger than the pre-tensioning force of the pre-tensioning element pre-tensioning the movable element 353 into its closed position. Thereby, it is ensured that the sealing member is open and can receive an inserting device.

The sealing member 350 of this embodiment may comprise or may be made from silicone, more particularly liquid silicone rubber.

The above embodiments according to Figures 3a to 3e preferably comprise a sealing member that is made from or comprises a material that is impermeable to liquids, specifically body liquids, like blood. Additionally or alternatively, the sealing members of the embodiments described so far may comprise or may be made of an electrically insulating material, so that flow of electrical currents through the receiving opening of the body part is prevented at least when the receiving opening and/or a hole in the sealing member caused by an inserting device is at least partially closed by the sealing member.

Figure 3f depicts a further embodiment of the sealing member 360. In this embodiment, the sealing member may be realized in line with any of the above embodiments according to Figures 3a to 3e.

Therefore, with respect to Figure 3f, further details regarding the characteristics of the sealing member 360 will not be provided but reference is made to the Figures 3a to 3e in this respect.

In addition to the embodiments described in relation to Figures 3a to 3e, the sealing member according to Figure 3f is made of or comprises an electrically conductive material 361. In the embodiment shown in Figure 3f, this electrically conductive material 361 is provided as a coating on the surface of the sealing member 360 that faces away from the skin 380 of the patient and may thus be considered to be at the "outer" surface of the sealing member 360. Instead of a coating, also embodiments where the electrically conductive material is directly included in the sealing member 360, or where the sealing member 360 is completely made from an electrically conductive material, are encompassed.

Providing the sealing member with an electrically conductive material allows for protecting the functional element 390 from electrical currents or voltages that can be caused, for example, when unintentionally contacting the body part 301 with an electrically charged object.

In order to conduct the current that is caused by this contact to a region of the body of the patient that is distant from the location where the functional element 390 is provided, an electrically conductive path 362 can be provided within the body part 301 or on or above the outer surface of the body part (and potentially electrically isolated from other components of the body part) so as to lead the current from the electrically conductive material 361 to the skin 380. The region of contact 363 of this conductive path 362 with the skin 380 of the patient can be spaced apart from the location where the functional element 390 is provided, for example at a distance of 1cm or 2cm. Any distance from the location where the functional element 390 is provided in the body of the patient can be thought of as long as the conductive path 362 contacts the skin 380 of the patient in the contacting region 363 that still is within the extension of the body part 301. Thereby the size of the distance of the functional element 390 to the contacting region 363 is limited by the physical extension of the body part. The minimum distance corresponds to the distance of the functional element to the border of the receiving opening and may already be sufficient so that in case the body part is at least partially electrically conductive, the electrically conductive material 361 contacting the body part 301 at the borders of the receiving opening can properly lead the electrical current to the region away from the functional element 390. In such a case, an electrically conductive part is not necessarily provided but the conducting of the current flow is ensured by the conductive material 361 and the body part itself, specifically the walls forming the borders of the receiving opening in the body part 301.

When providing the electrically conductive path 362, the flow of current to the contacting region 363 can be guided so that the contacting 363 region is further away from the receiving opening. This can increase the security of the functional element 390. The conductive path can, for example, be a cable made from aluminum or copper or other materials. Likewise, the electrically conductive material may comprise copper or aluminum or iron or other electrically conductive metals.

As already mentioned above, such electrically conductive material 361 as well as the embodiments described with respect to the conductive path 362 can be combined with all embodiments according to Figures 3a to 3e.

Additionally or alternatively, the embodiments described in relation to Figures 3a to 3f may comprise, as part of the material of the sealing member, a material or a plurality of materials that support the coagulation of blood. This can improve the capability of the sealing member to close the opening in the sealing member and thus also the receiving opening after retraction of an inserting device together with the coagulation of blood immediately below in the region of the wound caused by the inserting device.

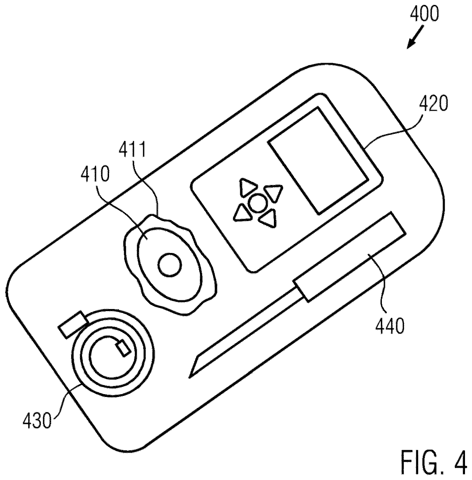

Figure 4 shows a kit 400 that can be provided to a patient for applying the devices and systems of the present disclosure. The kit 400 may comprise the analyte monitor 420. In other embodiments, the kit 400 does not comprise an analyte monitor 420 but the analyte monitor 420 may be provided to a patient separately.

The kit 400 may comprise an inserting device 440 for example in the form of a needle and either separately or already as part of the inserting device, the kit may comprise one or more functional elements 430, for example an analyte sensor, for subcutaneous implantation.

Additionally, the kit 400 may comprise a device comprising a body part 410 for application to the skin of the patient. Together with this body part 410, a plaster 411 or other fixation device for attaching the body part 410 to the skin of the patient may be provided. The fixation device 411 for attaching the body part 410 to the skin of the patient may be already prefixed to the body part 410 or may be provided separately, for example in a sterile sleeve.

The kit can be provided in a sterile packaging or at least the inserting device 440, the functional element 430 and the body part 410 can be sterilized before inclusion in the kit and can further be kept in the kit for example within a sterile sleeve until they are used by the patient in order to reduce infectious risks.

**List of reference signs**

| Reference sign | Element |
|---|---|
| 100 | Analyte monitoring system |
| 101 | Device |
| 102, 221 | Receiving opening |
| 110, 202, 262, 390, 430 | Functional element |
| 113, 213, 411 | Fixation device |
| 112, 201, 301, 410 | Body part |
| 120, 420 | Analyte monitor |
| 121 | Display |
| 122 | Keys |
| 123 | Connection |
| 130 | Patient |
| 230, 380 | Skin |
| 240, 260, 440 | Inserting device |
| 263 | Guiding needle |
| 264 | Tip |
| 251 | Upper surface |
| 252 | Region |
| 222, 310, 320, 330, 340, 350, 360 | Sealing member |
| 331, 332, 341, 342 | Remaining parts |
| 333, 334 | Reservoir |
| 343 | Blood |
| 351, 352 | Additional elements |
| 353 | Movable element |
| 354 | Pre-tensioning element |
| 361 | Electrically insulating material |
| 362 | Conductive path |
| 363 | Region of contact |
| 400 | Kit |

## Claims

1. A device (101) comprising a body part (112) that is attachable to the skin of a patient,
wherein the body part (112) comprises a receiving opening (102) through which a functional element (110) can be reversibly introduced into a body of a patient by means of an inserting device (240), like a needle, wherein the body part (112) comprises a sealing member (222) that is adapted to seal at least a part of the receiving opening (102) after removal of the inserting device (240), **characterized in that** the sealing member (360) is made from or comprises an electrically conductive material and the electrically conductive material is conductively connectable to the skin (230) of a patient when the device is attached to Z the skin of a patient.

2. The device according to claim 1, wherein the sealing member (222) comprises a self-sealing material that is adapted to seal or partially seal an opening in the self-sealing material that was caused by an inserting device (240), when or after the inserting device (240) is removed.

3. The device according to claim 2, wherein the self-sealing material comprises a foil (310) or a foam (320) or a preformed material.

4. The device according to claim 1, wherein the sealing member (222) comprises a sealing element (330) that can be introduced into the receiving opening (102).

5. The device according to claim 4, wherein the sealing element (330) comprises a stopper that can adapt to the form of the receiving opening (102).

6. The device according to claim 1, wherein the sealing member (350) comprises a movable element (353) that can be moved, by an inserting device, into an open position when the inserting device (240) is inserted through the receiving opening (102) and/or into a closed position when the inserting device (240) is retracted from the receiving opening (102).

7. The device according to claim 1, wherein the sealing member (340) comprises an absorbent element that is adapted to absorb a fluid and change, upon absorbing a fluid, its form so as to seal at least part of the receiving opening (102) after removal of an inserting device (240).

8. The device according to claim 7, wherein the absorbent material comprises one or more components that support coagulation of blood (343).

9. The device according to any of claims 1 to 8, wherein the sealing member (360) is made from or comprises an electrically insulating material (361) so that a flow of electrical current through the receiving opening (102) is prevented when the sealing member (360) seals at least a part of the receiving opening (102) after removal of an inserting device (240.

10. Combination of a functional element (110) and a device according to any of claims 1 to 9, wherein the functional element (110) is or comprises a subcutaneous sensor, a subcutaneous drug infusion cannula or a combined subcutaneous drug infusion cannula with a subcutaneous sensor.

11. An analyte monitoring system (100) comprising a transcutaneous sensor system and an analyte monitor (120) connected or connectable to the transcutaneous sensor system, the transcutaneous sensor system comprising a device (101) according to any of claims 1 to 9, a subcutaneous sensor (110) and a transmitter, wherein the subcutaneous sensor (110) is adapted to sense presence of at least one analyte and wherein the transmitter is adapted to transmit a signal indicative of a sensed analyte to the analyte monitor (120).

12. The analyte monitoring system (100) according to claim 11, wherein the analyte monitoring system (100) is a continuous analyte monitor and the subcutaneous sensor (110) is adapted to sense glucose in a body fluid of a patient when implanted.

13. A method of operating the device according to any of claims 1 to 9, comprising retracting an inserting device (240) from a receiving opening (221) of a body part (201) of a device, wherein the body part (201) is attached to the skin of a patient, wherein the method further comprises sealing at least part of the receiving opening (221) with a sealing member (222) of the body part after retracting the inserting device (240) from the receiving opening (221), wherein the sealing member (360) is made from or comprises an electrically conductive material and the electrically conductive material is conductively connectable to the skin (230) of a patient when the device is attached to the skin of a patient.

14. The method of claim 13, further comprising, before retracting the inserting device (240) from the receiving opening (221), inserting the inserting device (240) through the receiving opening (221) and placing a subcutaneous sensor into a region below the skin of the patient.

15. A kit (400) comprising a device (101) according to any of claims 1 to 9, a functional element (430) and an inserting device (440) for inserting the functional element (430) into a body of a patient.

16. The kit according to claim 15,
wherein the inserting device (440) is or comprises a needle.

## Patentansprüche

1. Vorrichtung (101), umfassend ein Körperteil (112), das auf der Haut eines Patienten anbringbar ist,
wobei das Körperteil (112) eine Aufnahmeöffnung (102) umfasst, durch die ein funktionelles Element (110) mittels einer Einführvorrichtung (240), wie einer Nadel, reversibel in einen Körper eines Patienten eingebracht werden kann, wobei das Körperteil (112) ein Abdichtbauteil (222) umfasst, das zum Abdichten mindestens eines Teils der Aufnahmeöffnung (102) nach der Entfernung der Einführvorrichtung (240) ausgelegt ist, **dadurch gekennzeichnet, dass** das Abdichtbauteil (360) aus einem elektrisch leitfähigen Material gefertigt ist oder ein solches umfasst und das elektrisch leitfähige Material leitfähig mit der Haut (230) eines Patienten verbunden werden kann, wenn die Vorrichtung auf der Haut eines Patienten angebracht wird.

2. Vorrichtung nach Anspruch 1, wobei das Abdichtbauteil (222) ein selbstabdichtendes Material umfasst, das zum Abdichten oder teilweisen Abdichten einer Öffnung in dem selbstabdichtenden Material, die von einer Einführvorrichtung (240) beim oder nach dem Entfernen der Einführvorrichtung (240) verursacht wurde, ausgelegt ist.

3. Vorrichtung nach Anspruch 2, wobei das selbstabdichtende Material eine Folie (310) oder einen Schaum (320) oder ein vorgeformtes Material umfasst.

4. Vorrichtung nach Anspruch 1, wobei das Abdichtbauteil (222) ein Abdichtelement (330) umfasst, das in die Aufnahmeöffnung (102) eingebracht werden kann.

5. Vorrichtung nach Anspruch 4, wobei das Abdichtelement (330) einen Stopper umfasst, der an die Form der Aufnahmeöffnung (102) angepasst werden kann.

6. Vorrichtung nach Anspruch 1, wobei das Abdichtbauteil (350) ein bewegliches Element (353) umfasst, das von einer Einführvorrichtung in eine offene Position, wenn die Einführvorrichtung (240) durch die Aufnahmeöffnung (102) eingeführt wird, und/oder in eine geschlossene Position, wenn die Einführvorrichtung (240) aus der Aufnahmeöffnung (102) herausgezogen wird, bewegt werden kann.

7. Vorrichtung nach Anspruch 1, wobei das Abdichtbauteil (340) ein absorptionsfähiges Element umfasst, das zum Absorbieren einer Flüssigkeit und nach dem Absorbieren einer Flüssigkeit zum Verändern seiner Form ausgelegt ist, sodass mindestens ein Teil der Aufnahmeöffnung (102) nach der Entfernung einer Einführvorrichtung (240) abgedichtet wird.

8. Vorrichtung nach Anspruch 7, wobei das absorptionsfähige Material eine oder mehrere Komponente(n) umfasst, die die Gerinnung von Blut (343) unterstützt/unterstützen.

9. Vorrichtung nach einem der Ansprüche 1 bis 8, wobei das Abdichtbauteil (360) aus einem elektrisch isolierenden Material (361) gefertigt ist oder ein solches umfasst, sodass ein Fluss von elektrischem Strom durch die Aufnahmeöffnung (102) verhindert wird, wenn das Abdichtbauteil (360) mindestens einen Teil der Aufnahmeöffnung (102) nach der Entfernung einer Einführvorrichtung (240) abdichtet.

10. Kombination aus einem funktionellen Element (110) und einer Vorrichtung nach einem der Ansprüche 1 bis 9, wobei das funktionelle Element (110) ein subkutaner Sensor, eine subkutane Arzneimittelinfusionskanüle oder eine kombinierte subkutane Arzneimittelinfusionskanüle mit einem subkutanen Sensor ist oder solche umfasst.

11. Analytüberwachungssystem (100), umfassend ein transkutanes Sensorsystem und ein Analytüberwachungsgerät (120), das an das transkutane Sensorsystem angeschlossen ist oder angeschlossen werden kann, wobei das transkutane Sensorsystem eine Vorrichtung (101) nach einem der Ansprüche 1 bis 9, einen subkutanen Sensor (110) und einen Sender umfasst, wobei der subkutane Sensor (110) zum Erfassen der Gegenwart mindestens eines Analyten ausgelegt ist und wobei der Sender zum Senden eines Signals, das einen erfassten Analyten kennzeichnet, an das Analytüberwachungsgerät (120) ausgelegt ist.

12. Analytüberwachungssystem (100) nach Anspruch 11, wobei das Analytüberwachungssystem (100) ein kontinuierliches Analytüberwachungsgerät ist und der subkutane Sensor (110) zum Erfassen von Glukose in einer Körperflüssigkeit eines Patienten ausgelegt ist, wenn er implantiert ist.

13. Verfahren zum Betreiben der Vorrichtung nach einem der Ansprüche 1 bis 9, umfassend das Herausziehen einer Einführvorrichtung (240) aus einer Aufnahmeöffnung (221) eines Körperteils (201) einer Vorrichtung, wobei das Körperteil (201) auf der Haut eines Patienten angebracht ist, wobei das Verfahren ferner das Abdichten mindestens eines Teils der Aufnahmeöffnung (221) mit einem Abdichtbauteil (222) des Körperteils nach dem Herausziehen der Einführvorrichtung (240) aus der Aufnahmeöffnung (221) umfasst, wobei das Abdichtbauteil (360) aus einem elektrisch leitfähigen Material gefertigt ist oder ein solches umfasst und das elektrisch leitfähige Material leitfähig mit der Haut (230) eines Patienten verbunden werden kann, wenn die Vorrichtung auf der Haut eines Patienten angebracht wird.

14. Verfahren nach Anspruch 13, ferner umfassend vor dem Herausziehen der Einführvorrichtung (240) aus der Aufnahmeöffnung (221) das Einführen der Einführvorrichtung (240) durch die Aufnahmeöffnung (221) und Platzieren eines subkutanen Sensors in eine Region unter der Haut des Patienten.

15. Kit (400), umfassend eine Vorrichtung (101) nach einem der Ansprüche 1 bis 9, ein funktionelles Element (430) und eine Einführvorrichtung (440) zum Einführen des funktionellen Elements (430) in einen Körper eines Patienten.

16. Kit nach Anspruch 15, wobei die Einführvorrichtung (440) eine Nadel ist oder umfasst.

## Revendications

1. Dispositif (101) comprenant une partie de corps (112) pouvant être fixée à la peau d'un patient, dans lequel la partie de corps (112) comprend une ouverture de réception (102) à travers laquelle un élément fonctionnel (110) peut être introduit de manière réversible dans un corps d'un patient au moyen d'un dispositif d'insertion (240), tel qu'une aiguille, dans lequel la partie de corps (112) comprend un membre obturant (222) qui est adapté pour obturer au moins une partie de l'ouverture de réception (102) après le retrait du dispositif d'insertion (240), **caractérisé en ce que** le membre obturant (360) est fabriqué à partir de ou comprend un matériau électriquement conducteur et le matériau électriquement conducteur peut être connecté de manière conductrice à la peau (230) d'un patient lorsque le dispositif est fixé à la peau d'un patient.

2. Dispositif selon la revendication 1, dans lequel le membre obturant (222) comprend un matériau auto-obturant qui est adapté pour obturer ou partiellement obturer une ouverture dans le matériau auto-obturant qui a été provoquée par un dispositif d'insertion (240), lorsque ou après que le retrait dispositif d'insertion (240).

3. Dispositif selon la revendication 2, dans lequel le matériau auto-obturant comprend une feuille (310) ou une mousse (320) ou un matériau préformé.

4. Dispositif selon la revendication 1, dans lequel le membre obturant (222) comprend un élément obturant (330) qui peut être introduit dans l'ouverture de réception (102).

5. Dispositif selon la revendication 4, dans lequel l'élément obturant (330) comprend un bouchon qui peut s'adapter à la forme de l'ouverture de réception (102).

6. Dispositif selon la revendication 1, dans lequel le membre obturant (350) comprend un élément déplaçable (353) qui peut être déplacé, par un dispositif d'insertion, dans une position ouverte lorsque le dispositif d'insertion (240) est inséré à travers l'ouverture de réception (102) et/ou dans une position fermée lorsque le dispositif d'insertion (240) est rétracté de l'ouverture de réception (102).

7. Dispositif selon la revendication 1, dans lequel le membre obturant (340) comprend un élément absorbant qui est adapté pour absorber un fluide et changer, lors de l'absorption d'un fluide, de forme de manière à obturer au moins une partie de l'ouverture de réception (102) après le retrait d'un dispositif d'insertion (240).

8. Dispositif selon la revendication 7, dans lequel le matériau absorbant comprend un ou plusieurs composants favorisant la coagulation du sang (343).

9. Dispositif selon l'une quelconque des revendications 1 à 8, dans lequel le membre obturant (360) est fabriqué à partir de ou comprend un matériau électriquement isolant (361) de sorte qu'un flux de courant électrique à travers l'ouverture de réception (102) est empêché lorsque le membre obturant (360) obture au moins une partie de l'ouverture de réception (102) après le retrait d'un dispositif d'insertion (240).

10. Combinaison d'un élément fonctionnel (110) et d'un dispositif selon l'une quelconque des revendications 1 à 9, dans laquelle l'élément fonctionnel (110) est ou comprend un capteur sous-cutané, une canule de perfusion de médicament sous-cutanée ou une canule de perfusion de médicament sous-cutanée combinée à un capteur sous-cutané.

11. Système de surveillance d'analyte (100) comprenant un système de capteur transcutané et un dispositif de surveillance d'analyte (120) connecté ou pouvant être connecté au système de capteur transcutané, le système de capteur transcutané comprenant un dispositif (101) selon l'une quelconque des revendications 1 à 9, un capteur sous-cutané (110) et un transmetteur, dans lequel le capteur sous-cutané (110) est adapté pour détecter la présence d'au moins un analyte et dans lequel le transmetteur est adapté pour transmettre un signal indicatif d'un analyte détecté au dispositif de surveillance d'analyte (120).

12. Système de surveillance d'analyte (100) selon la revendication 11, dans lequel le système de surveillance d'analyte (100) est un dispositif de surveillance d'analyte en continu et le capteur sous-cutané (110) est adapté pour détecter le glucose dans un fluide corporel d'un patient lorsqu'il est implanté.

13. Procédé de fonctionnement du dispositif selon l'une quelconque des revendications 1 à 9, comprenant la rétractation d'un dispositif d'insertion (240) d'une ouverture de réception (221) d'une partie de corps (201) d'un dispositif, dans lequel la partie de corps (201) est fixée à la peau d'un patient, dans lequel le procédé comprend en outre l'obturation d'au moins une partie de l'ouverture de réception (221) avec un membre obturant (222) de la partie de corps après la rétractation du dispositif d'insertion (240) de l'ouverture de réception (221), dans lequel le membre obturant (360) est fabriqué à partir de ou comprend un matériau électriquement conducteur et le matériau électriquement conducteur peut être connecté de manière conductrice à la peau (230) d'un patient lorsque le dispositif est fixé à la peau d'un patient.

14. Procédé selon la revendication 13, comprenant en outre, avant la rétractation du dispositif d'insertion (240) de l'ouverture de réception (221), l'insertion du dispositif d'insertion (240) à travers l'ouverture de réception (221) et la mise en place d'un capteur sous-cutané dans une région sous la peau du patient.

15. Kit (400) comprenant un dispositif (101) selon l'une quelconque des revendications 1 à 9, un élément fonctionnel (430) et un dispositif d'insertion (440) pour insérer l'élément fonctionnel (430) dans un corps d'un patient.

16. Kit selon la revendication 15, dans lequel le dispositif d'insertion (440) est ou comprend une aiguille.
